# FASCICULE DE BREVET EUROPEEN

(11) **EP 3 105 186 B1**
(45) Date de publication et mention de la délivrance du brevet: **04.04.2018**
(21) Numéro de dépôt: 15709747.8
(22) Date de dépôt: 12.02.2015
(51) Int. Cl.: B05B 15/04, F23L 17/16, A61L 9/00

(54) **DISPOSITIF POUR DISPERSER A LA SOURCE LES EMISSIONS D'EFFLUENTS GAZEUX NAUSEABONDS**
VORRICHTUNG ZUM DISPERGIEREN VON STINKENDEN ABGASEMISSIONEN AN DER QUELLE
DEVICE FOR DISPERSING FOUL GASEOUS EFFLUENT EMISSIONS AT THE SOURCE

(30) Priorité: 14.02.2014 FR 1451164
(43) Date de publication de la demande: 21.12.2016
(73) Titulaire: SUEZ Groupe, 92040 Paris la Défense Cedex (FR)
(72) Inventeur: DUONG, Frédéric, F-66370 Pezilla-la-RIivière (FR); KELLY, Robert, F-78670 Medan (FR); CHANTRE, Patrick, F-34420 Portiragnes (FR)
(74) Mandataire: Cabinet Armengaud Aîné
(86) Numéro de dépôt international: PCT/IB2015/051052
(87) Numéro de publication internationale: WO 2015/121820

(56) Documents cités:
- EP-A1- 0 772 003
- EP-A1- 2 161 446
- WO-A1-2010/044055

## Description

L'invention est relative à un dispositif de dispersion à la source des émissions d'effluents gazeux nauséabonds, dégagés de manière diffuse par des sources situées à proximité du sol, en particulier par des bassins de traitement d'eaux usées ou toute autre activité qui dégage des odeurs.

De nombreuses activités industrielles émettent des odeurs nauséabondes qui se dispersent au gré du vent. Ces effluves peuvent générer une gêne importante pour les riverains. Les émissions d'odeur sont difficilement contrôlables, en particulier lorsqu'il n'est pas possible de les confiner dans une enceinte étanche, et lorsque les surfaces d'émission sont importantes et à géométrie variable.

Les odeurs ressenties sont relatives à la concentration et à la nature chimique des molécules produites au niveau de la source d'odeurs. De nombreux facteurs météorologiques influent sur les émissions et surtout sur la dispersion des odeurs au niveau de l'environnement. Il s'agit en particulier de :
- la vitesse et du sens du vent,
- l'humidité et la température de l'air ambiant,
- la pression atmosphérique,
- l'effet du relief et des bâtiments avoisinants qui peuvent produire des courants rabattants.

Divers moyens de réduction de l'impact des nuisances olfactives ont été proposés.

### Cas de flux canalisés

Afin de favoriser la dispersion des gaz ou de l'air nauséabond, après un traitement chimique ou biologique, les effluents gazeux peuvent être dispersés à une certaine hauteur dans l'atmosphère par des cheminées. La hauteur des cheminées est variable selon la nature et la composition des gaz, la réglementation en vigueur et les résultats de l'étude de dispersion des polluants gazeux, particulaires et malodorants.

La dispersion des gaz dans l'environnement est améliorée en augmentant la hauteur de cheminée. Un régime de vents turbulents et soutenus a pour effet de mélanger les gaz avec l'air ambiant et de favoriser ainsi la dispersion des polluants.

L'effet de dispersion des polluants qui est lié à la hauteur d'éjection des gaz dans l'atmosphère est parfois annihilé par des courants d'air (vents) rabattants. Ces conditions défavorables peuvent entraîner une concentration de polluants ou d'odeurs au niveau du sol supérieure au seuil maximal admissible ressenti, ceci malgré le respect de la réglementation concernant les rejets de polluants en sortie des exutoires. EP 2161446 décrit une éolienne qui favorise la dispersion de gaz dans l'atmosphère à proximité d'une station d'épuration d'eaux résiduaires et qui comprend un mât vertical équipé d'un rotor avec pales, le mât pouvant ainsi servir pour l'évacuation de gaz. WO 2010/044 055 a proposé d'améliorer et de favoriser la dispersion des gaz en prévoyant au moins un injecteur d'air ou de gaz auxiliaire installé à proximité du débouché d'une cheminée et orienté vers le haut de manière à disperser les gaz.

### Cas de flux diffus

Les émissions peuvent être diffuses et provenir, par exemple, de grands bassins de stations d'épuration, de centres d'enfouissement, de zones de stockage de déchets organiques, d'aires de maturation de matières organiques et autres installations similaires. De telles installations ne sont pas nécessairement à ciel ouvert, et peuvent être recouvertes au moins en partie, mais les effluents gazeux ne sont plus canalisés vers un exutoire, notamment une cheminée. Il n'est alors plus possible ou difficile et coûteux de capter à la source les effluves nauséabonds, et un moyen utilisé pour lutter contre les odeurs consiste à masquer ces odeurs en diffusant des produits "blancs" dont l'objectif est de réduire l'impact olfactif, en couvrant les odeurs.

Un tel moyen présente une efficacité très limitée car il n'agit que localement dans la zone de diffusion des produits masquants. De plus, les molécules utilisées ne sont pas totalement neutres pour l'environnement.

### Mesure des unités d'odeur

Afin d'évaluer d'une manière objective le taux d'odeur ambiant, des capteurs électrochimiques ont été mis au point. De tels capteurs sont positionnés dans les zones de présence humaine pour mesurer en continu la concentration en molécules les plus odorantes telles que H₂S, NH₃, mercaptan. Ces capteurs permettent de convertir les mesures chimiques en unités d'odeur qui font référence à la perception odorante humaine, et d'indiquer le facteur de nuisance en cours.

Ces capteurs sont utiles car ils permettent de prévenir les riverains d'un pic d'émission et de prévenir le public avant un constat *a posteriori* qui entraîne de nombreuses plaintes. Toutefois, ces mesures d'odeur n'ont pas d'effet correctif sur les émissions ni sur leur perception.

L'invention a pour but, surtout, de fournir un dispositif qui permet d'améliorer significativement la dispersion des effluents gazeux nauséabonds dégagés de manière diffuse par des installations qui ne permettent pas de canaliser ces effluents. Le dispositif selon l'invention est un dispositif de dispersion à la source des émissions d'effluents gazeux nauséabonds, caractérisé en ce qu'il comporte :
- un bassin source d'effluents gazeux nauséabonds,
- au moins deux injecteurs (5a, 5b) d'air ou de gaz auxiliaire orientés vers le haut, installés à la périphérie du bassin source d'effluents gazeux nauséabonds, dans des zones opposées, de manière à disperser les effluents gazeux nauséabonds,
- des moyens d'alimentation des injecteurs, chaque injecteur étant alimenté par un moyen d'alimentation séparé,
- et des moyens de commande sensibles au sens du vent agencés pour sélectionner la mise en fonctionnement du ou des injecteur(s) dont la situation, selon le sens du vent, est la plus favorable pour assurer la dispersion et/ou la remontée du panache d'effluents gazeux.

De préférence, le dispositif comporte des détecteurs d'odeurs répartis à la périphérie d'une installation comportant au moins une source d'odeurs nauséabondes, ces détecteurs étant agencés pour communiquer les résultats de leurs mesures à une centrale d'acquisition de mesures reliée à une unité de commande qui, selon les résultats des mesures, met en action au moins un ventilateur pour alimenter au moins un secteur approprié d'injecteurs.

Généralement, les injecteurs sont situés à une hauteur inférieure à celle du bord supérieur de la source d'odeurs, en particulier en bordure de la zone d'émission d'odeurs, c'est-à-dire à proximité du bord supérieur de la source d'odeurs.

Avantageusement, le dispositif comporte au moins trois secteurs d'injecteurs installés à la périphérie d'une, ou de chaque, source d'odeurs nauséabondes, chaque secteur comportant au moins un injecteur.

Chaque secteur angulaire et ses injecteurs, sont alimentés par une rampe de distribution d'air équipée d'une vanne à commande motorisée, la rampe étant reliée au refoulement d'un ventilateur.

De préférence, les injecteurs sont à orientation réglable.

Les moyens d'alimentation des injecteurs et les moyens de commande peuvent être prévus pour assurer une pression variable en fonction des conditions atmosphériques et de mesures d'unités d'odeur.

Avantageusement, l'unité de commande est pilotée par un logiciel de gestion et de contrôle programmé pour prendre en considération les paramètres de dispersion et les mesures d'unités d'odeur en temps réel.

L'unité de commande peut être pilotée par un logiciel de simulation prédictif de la dispersion naturelle du panache d'odeurs, intégré pour une action préventive de commande automatique du dispositif de dispersion par induction.

Le dispositif peut comporter un moyen d'injection de produits « masquants » dans l'air qui alimente les injecteurs.

Avantageusement, les moyens d'alimentation des injecteurs comprennent au moins un ventilateur à vitesse réglable, et au moins une conduite de liaison entre la sortie du ventilateur et une rampe d'alimentation du ou des injecteurs.

De préférence, les moyens d'alimentation du ou des injecteurs sont réglables pour permettre de moduler la pression et le débit d'air ou de gaz auxiliaire envoyé aux injecteurs selon les conditions atmosphériques du moment, et les propriétés du panache de gaz, l'arrêt des moyens d'alimentation étant commandé lorsque la dispersion naturelle est suffisante ou les conditions pour lesquelles les émissions d'odeurs sont faibles.

L'invention consiste, mises à part les dispositions évoquées ci-dessus, en un certain nombre d'autres dispositions dont il sera plus explicitement question ci-après à propos d'exemples décrits avec référence aux dessins annexés, mais qui ne sont nullement limitatifs. Sur ces dessins :
Fig. 1 est un schéma illustrant un bassin de traitement d'eau selon l'état de la technique.
Fig. 2 est un schéma, semblable à celui de Fig. 1, du bassin avec dispositif selon l'invention pour disperser les émissions d'effluents gazeux, et
Fig. 3 est un schéma d'une usine de traitement d'eaux usées dont les différentes sources d'effluents gazeux nauséabonds sont équipées de dispositifs selon l'invention.

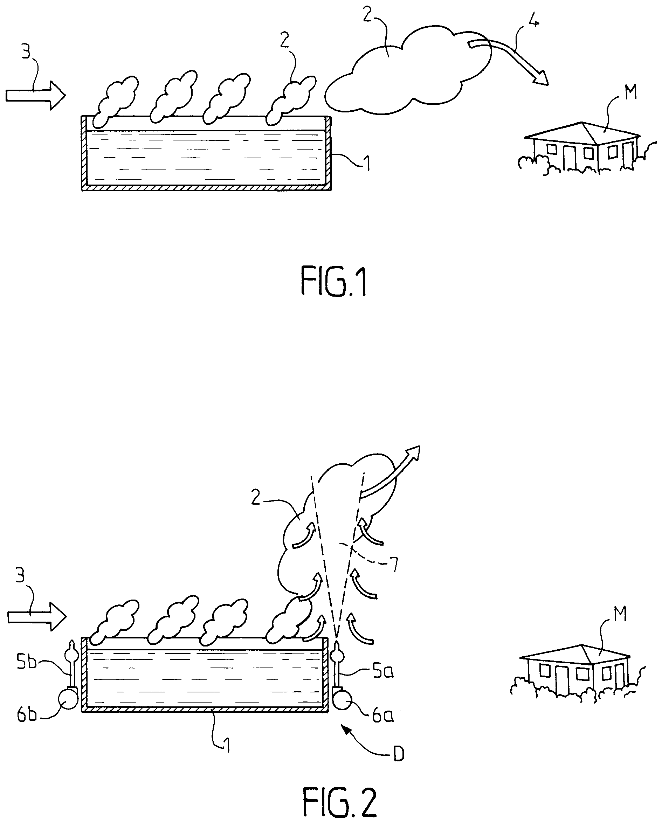

En se reportant à Fig. 1 des dessins, on peut voir un bassin 1 de traitement d'eaux usées, selon l'état de la technique. Un tel bassin est généralement circulaire, et son diamètre peut atteindre 30 m et plus. Le bassin 1 dégage des panaches 2 d'effluents gazeux nauséabonds.

Le volume des panaches 2 dépend essentiellement de l'évaporation et donc de paramètres constitués par la vitesse de l'air de balayage en surface du bassin, la température ambiante, l'hygrométrie et la pression atmosphérique. Les panaches 2 sont poussés par le vent dont le sens est schématisé par une flèche 3 et peuvent atteindre des habitations M, qui se trouvent du côté du bassin opposé au sens du vent. Leurs habitants vont ainsi être incommodés par les effluents dégagés. Cette gêne sera d'autant plus forte dans le cas de vents rabattants, illustrés par la flèche 4 sur Fig. 1, en direction des habitations.

Afin d'augmenter la dispersion des odeurs nauséabondes dans l'air ambiant, un dispositif D (Fig.2) selon l'invention comporte au moins deux injecteurs 5a, 5b d'air, ou de gaz auxiliaire, orientés vers le haut et constituant des inducteurs pour disperser les effluents gazeux. Les injecteurs 5a, 5b sont alimentés en air à moyenne pression, notamment comprise entre 50 daPa et 300 daPa, par des rampes 6a, 6b branchées sur le refoulement d'un ventilateur non visible sur Fig.2. Les rampes ou conduites d'alimentation, telles que 6a, 6b sont avantageusement réalisées en tôle spiralée ou en autre matériau léger (notamment textile).

Les injecteurs 5a, 5b sont placés en périphérie de chaque source d'émission d'odeurs, de manière à assurer une dispersion rapide des odeurs par leur dilution dans l'air ambiant. Les injecteurs 5a, 5b sont situés à proximité du sol, à une hauteur voisine de la zone d'émission. L'induction créée par un jet d'air 7 orienté vers le haut favorise l'ascension dynamique et la dispersion du panache malodorant 2 lorsque les effets d'un vent rabattant sont supérieurs à ceux dus à la différence de densité entre les gaz et l'air ambiant.

Les rampes 6a, 6b constituent des moyens d'alimentation séparés des injecteurs. Ainsi, chaque injecteur 5a, 5b est alimenté par un moyen d'alimentation séparé.

La distribution d'air moteur vers les injecteurs 5a, 5b peut ainsi être fractionnée de manière à permettre de sélectionner le ou les injecteurs à mettre en fonctionnement selon le sens du vent et la localisation d'émission des odeurs. Selon le schéma de Fig. 2, il suffit de faire fonctionner l'injecteur 5a situé du côté du bassin 1 opposé au sens du vent 3, pour obtenir un effet satisfaisant.

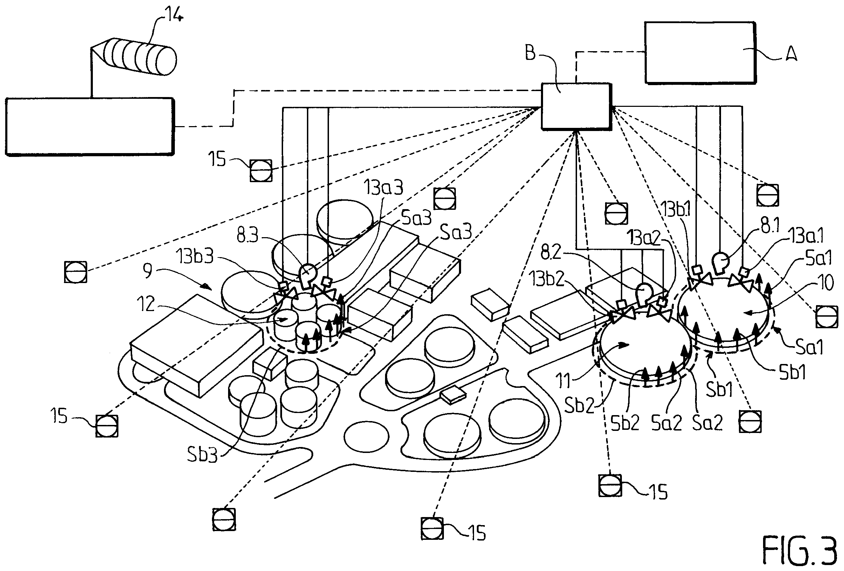

Avantageusement, comme illustré sur Fig. 3, des moyens de commande B sensibles au sens du vent et à différents paramètres météorologiques, sont prévus pour assurer l'alimentation du ou des injecteur(s) dont la position est la plus favorable pour assurer la dispersion et la remontée du panache d'effluents gazeux.

Ainsi, les moyens de commande B sensibles au sens du vent sont agencés pour sélectionner la mise en fonctionnement du ou des injecteur(s) 5a, 5b dont la situation, selon le sens du vent, est la plus favorable pour assurer la dispersion et/ou la remontée du panache d'effluents gazeux.

La pression en sortie des jets d'air est variable et réglable de manière à adapter en permanence le taux d'induction, ou taux d'entraînement du panache par le jet 7 dirigé vers le haut, par rapport aux besoins et afin de limiter la consommation d'énergie, essentiellement électrique, du dispositif d'alimentation en air moteur. Ce dispositif d'alimentation comporte, pour chaque source d'odeurs, au moins un ventilateur 8.1, 8.2, 8.3 (Fig. 3) moyenne pression, notamment de 50 daPa à 300daPa, à vitesse variable, réglable.

Fig. 3 montre une usine 9 de traitement d'eaux usées, équipée de dispositifs selon l'invention avec plusieurs injecteurs 5a1... 5b3 installés en périphérie de zones d'émission d'odeurs constituées de bassins 10, 11, et d'une zone 12 de cuves de traitement, ou de surfaces de stockage de déchets et de maturation de matières organiques ou autres. Au niveau de chaque source d'effluves malodorants, les injecteurs 5a1, ...5b3 sont répartis en au moins deux secteurs Sa1, Sb1...Sa3, Sb3 autour de la source. Chaque secteur est alimenté indépendamment en air sous pression par une rampe dont le débit est contrôlé par un registre ou une vanne motorisée 13a1 ... 13b3, située de part et d'autre d'une injection d'air provenant d'un ventilateur 8.1, 8 .2, 8.3.

Pour le bassin 10, deux secteurs Sa1, Sb1 d'injecteurs 5a1, 5b1 sont prévus autour du bassin. Chaque secteur s'étend suivant sensiblement un demi-cercle et est alimenté respectivement, par l'intermédiaire d'un registre ou d'une vanne automatisée 13a1, 13b1, par un ventilateur 8.1 dont le refoulement est branché sur une partie de conduite comprise entre les vannes 13a1 et 13b1 contrôlant le débit vers les secteurs Sa1, Sb1.

Les injecteurs 5a1, 5b1, représentés en nombre réduit pour simplifier sur Fig. 3, sont de préférence répartis régulièrement à la périphérie de la source d'odeurs constituée par le bassin 10. La distance entre deux injecteurs voisins peut être de l'ordre d'au moins 1 m, et peut atteindre 5 m et plus selon le périmètre à traiter. Le sens du vent détecté par une manche à air 14 et la force du vent détectée par un anémomètre (non représenté) sont transmis à une centrale d'acquisition de mesures B, et l'alimentation en air sous pression est dirigée vers le secteur situé du côté du bassin opposé au sens du vent.

Des dispositions semblables sont adoptées pour le bassin 11 et pour la source d'émission 12, les éléments identiques étant désignés par les mêmes références affectées de 2 ou 3 comme dernier chiffre.

Le nombre de secteurs d'injecteurs disposés à la périphérie d'une source peut être supérieur à deux afin de mieux prendre en compte le sens du vent et de limiter le nombre d'injecteurs par secteur. Avantageusement, chaque secteur d'injecteurs est alimenté séparément.

Les jets d'air 7 orientés vers le haut permettent d'éclater le flux de gaz nauséabonds et de disperser rapidement le panache d'odeurs pendant les périodes de vents rabattants.

Le réglage de la pression d'air est assuré par un variateur de vitesse du moteur du ventilateur 8.1, 8.2, 8.3 concerné, ou par tout autre moyen mécanique, notamment un registre.

La pression d'air au niveau des injecteurs est ajustée par la centrale B, généralement entre 50 daPa et 300daPa, selon les conditions atmosphériques, sens et intensité du vent, pression et température externe. Les injecteurs peuvent être totalement arrêtés lorsque les conditions météorologiques sont favorables à la dispersion naturelle des odeurs, notamment par grand vent, ou au contraire par calme total avec pression atmosphérique élevée.

Des capteurs d'odeurs 15, notamment électrochimiques, sont répartis à une distance appropriée tout autour de l'usine 9 pour effectuer une mesure en continu de la concentration en molécules les plus malodorantes, telles que H₂S, NH₃, mercaptan. Les résultats des mesures ainsi réalisées sont transmis à la centrale B d'acquisition des différents paramètres et des mesures météorologiques. Les données recueillies par la centrale B sont transmises à une unité A constituée par un ordinateur équipé d'un programme de gestion et de commande automatique des différentes fonctions. Un écran numérique, non représenté, permet d'indiquer le déplacement du panache d'odeurs, les mesures et les équipements de dispersion en action. La centrale B commande les actionneurs des vannes 13a1, 13b1... 13b3 pour régler leur fonctionnement, notamment au niveau du débit.

La disposition des capteurs d'odeurs 15 autour de l'usine 9 permet d'agir en temps réel et de moduler le fonctionnement du dispositif de dispersion des effluents malodorants par induction en prenant en compte les conditions météorologiques du site : vitesse et sens du vent, température et humidité de l'air, pression atmosphérique. Les mesures fournies par les capteurs 15 permettent de corriger les paramètres de fonctionnement et de vérifier l'efficacité du dispositif.

Le fonctionnement du dispositif pour lutter contre les émissions d'effluents gazeux malodorants résulte des explications qui précèdent.

Si les informations fournies par les capteurs d'odeurs 15 ne font pas apparaître un niveau d'odeur dépassant un seuil déterminé, la centrale B maintient les ventilateurs 8.1, 8.2, 8.3 à l'arrêt et les vannes 13a1, 13b1 ... 13a3, 13b3 en position de fermeture.

Si les capteurs 15 signalent un dépassement du seuil déterminé dans une zone, généralement située du côté de l'usine opposé au sens du vent, la centrale B commande la mise en marche de l'un au moins des ventilateurs 8.1, 8.2, 8.3, et l'ouverture de la vanne appropriée 13a1...13b3, pour que les injecteurs appropriés soient mis en fonctionnement.

Les capteurs 15 permettent de vérifier si l'efficacité de dispersion est suffisante pour que le niveau d'odeur baisse au-dessous du seuil déterminé.

Pour le cas où le niveau d'odeur ne pourrait être ramené à une valeur inférieure au seuil tolérable, un opérateur préviendra le personnel de l'usine et les riverains, ou fera arrêter, dans la mesure du possible, les sources d'effluents gazeux malodorants.

Le dispositif avec injecteurs d'air selon l'invention permet d'assurer deux effets : une dilution du panache d'effluents gazeux malodorants et un effet de montée de ce panache.

Des produits masquants peuvent être injectés simultanément dans l'air qui alimente les injecteurs 5a1... 5b3.

Un logiciel de modélisation de dispersion des diverses sources d'effluents gazeux malodorants est avantageusement installé dans l'unité A pour permettre d'anticiper l'intensité de formation d'effluents gazeux malodorants, et la formation de panaches selon les conditions météorologiques. Il est alors possible d'agir préventivement en faisant fonctionner les secteurs d'injecteurs appropriés.

Le dispositif selon l'invention concerne non seulement des installations à ciel ouvert, mais aussi les installations de type semi-fermées, c'est-à-dire celles qui comportent une couverture avec des orifices de sortie dépourvus de hautes cheminées. Il s'agit notamment de serres pour le séchage de boues de stations d'épuration ; de telles serres comportent une toiture dans laquelle sont prévues des ouvertures, pour l'évacuation des effluents. Selon l'invention, des injecteurs d'air pourraient être répartis autour d'une serre pour disperser les effluents qui s'échappent par les ouvertures du toit.

L'invention présente de nombreux avantages.

L'asservissement automatique du dispositif de dispersion d'odeurs par induction, commandé par le système de contrôle comportant les capteurs d'odeurs 15, permet de contrôler et de maîtriser le niveau d'unités d'odeur en périphérie de l'usine 9, et également d'optimiser en permanence le fonctionnement du dispositif à la fois préventivement et en action corrective selon les différents paramètres mesurés.

La consommation d'énergie du dispositif est relativement faible par rapport à l'effet de dilution important avec l'air ambiant. Le dispositif est régulé automatiquement afin d'obtenir l'effet de dispersion maximal tout en limitant sa consommation d'énergie électrique.

Le dispositif est simple à mettre en oeuvre et peu coûteux au regard d'une installation de captation et de traitement chimique biologique des effluents gazeux malodorants. Le dispositif peut être installé sur des unités neuves ou existantes.

L'invention s'applique à toute installation à protéger contre les nuisances olfactives diffuses, notamment
- bassins d'épaississement ou d'aération de STEP (stations d'épuration d'eaux usées),
- centres de stockage des déchets,
- plates-formes de compostage de matières organiques,
- centres de transfert de déchets,
- toute zone d'activité industrielle ouverte émettant des odeurs nauséabondes.

## Revendications

1. Dispositif de dispersion à la source des émissions d'effluents gazeux nauséabonds, **caractérisé en ce qu'**il comporte :
- un bassin (1) source d'effluents gazeux nauséabonds,
- au moins deux injecteurs (5a, 5b) d'air ou de gaz auxiliaire orientés vers le haut, installés à la périphérie du bassin (1) source d'effluents gazeux nauséabonds, dans des zones opposées, de manière à disperser les effluents gazeux nauséabonds,
- des moyens d'alimentation (6a, 6b) des injecteurs, chaque injecteur étant alimenté par un moyen d'alimentation séparé,
- et des moyens de commande sensibles au sens du vent agencés pour sélectionner la mise en fonctionnement du ou des injecteur(s) dont la situation, selon le sens du vent, est la plus favorable pour assurer la dispersion et/ou la remontée du panache d'effluents gazeux.

2. Dispositif selon la revendication 1, **caractérisé en ce qu'**il comporte des détecteurs d'odeurs (15) répartis à la périphérie d'une installation comportant au moins une source (10,11,12) d'odeurs nauséabondes, ces détecteurs étant agencés pour communiquer les résultats de leurs mesures à une centrale (B) d'acquisition de mesures reliée à une unité de commande (A) qui, selon les résultats des mesures, met en action au moins un ventilateur (8.1,8.2,8.3) pour alimenter au moins un secteur approprié (Sa1...Sb3) d'injecteurs.

3. Dispositif selon la revendication 1 ou 2, **caractérisé en ce que** les injecteurs (5a, 5b) sont situés à proximité du bord supérieur de la source d'odeurs,

4. Dispositif selon l'une quelconque des revendications précédentes, **caractérisé en ce qu'**il comporte au moins trois secteurs d'injecteurs installés à la périphérie d'une, ou de chaque, source d'odeurs nauséabondes, chaque secteur comportant au moins un injecteur.

5. Dispositif selon l'une quelconque des revendications précédentes, **caractérisé en ce que** chaque secteur angulaire (Sa1,...Sb3), et ses injecteurs, sont alimentés par une rampe de distribution d'air équipée d'une vanne à commande motorisée (13a1,...13b3), la rampe étant reliée au refoulement d'un ventilateur (8a1, 8a2, 8a3).

6. Dispositif selon l'une quelconque des revendications précédentes, **caractérisé en ce que** les injecteurs (5a, 5b) sont à orientation réglable.

7. Dispositif selon l'une quelconque des revendications précédentes, **caractérisé en ce que** les moyens d'alimentation (6a, 6b) des injecteurs et les moyens de commande sont prévus pour assurer une pression variable en fonction des conditions atmosphériques et de mesures d'unités d'odeur.

8. Dispositif selon la revendication 2, **caractérisé en ce que** l'unité de commande (A) est pilotée par un logiciel de gestion et de contrôle programmé pour prendre en considération les paramètres de dispersion et les mesures d'unités d'odeur en temps réel.

9. Dispositif selon la revendication 2 ou 8, **caractérisé en ce que** l'unité de commande (A) est pilotée par un logiciel de simulation prédictif de la dispersion naturelle du panache d'odeurs (2), intégré pour une action préventive de commande automatique du dispositif de dispersion par induction.

10. Dispositif selon l'une quelconque des revendications précédentes, **caractérisé en ce qu'**il comporte un moyen d'injection de produits « masquants » dans l'air qui alimente les injecteurs.

11. Dispositif selon l'une quelconque des revendications précédentes, **caractérisé en ce que** les moyens d'alimentation des injecteurs comprennent au moins un ventilateur (8.1, 8.2, 8.3) à vitesse réglable, et au moins une conduite de liaison entre la sortie du ventilateur et une rampe d'alimentation du ou des injecteurs.

12. Dispositif selon la revendication 11, **caractérisé en ce que** les moyens d'alimentation du ou des injecteurs sont réglables pour permettre de moduler la pression et le débit d'air ou de gaz auxiliaire envoyé aux injecteurs selon les conditions atmosphériques du moment, et les propriétés du panache de gaz, l'arrêt des moyens d'alimentation étant commandé lorsque la dispersion naturelle est suffisante.

## Patentansprüche

1. Vorrichtung zum Dispergieren von übelriechenden Abgasemissionen an der Quelle, **dadurch gekennzeichnet, dass** sie aufweist:
- ein Becken (1) als Quelle übelriechender Abgase,
- mindestens zwei nach oben gerichtete Hilfsgas- oder Hilfsluftinjektoren (5a, 5b), welche am Umfang des die Quelle übelriechender Abgase bildenden Beckens (1) in gegenüberliegenden Zonen derart installiert sind, dass sie die übelriechenden Abgase verteilen,
- Einrichtungen (6a, 6b) zur Versorgung der Injektoren, wobei jeder Injektor durch eine separate Versorgungseinrichtung versorgt wird,
- und für die Windrichtung empfindliche Steuereinrichtungen, welche derart angeordnet sind, dass sie die Inbetriebnahme des Injektors oder der Injektoren wählen, dessen/deren Position entsprechend der Windrichtung die günstigste ist, um das Dispergieren und/oder das Aufsteigen der Abgaswolke zu gewährleisten.

2. Vorrichtung nach Anspruch 1, **dadurch gekennzeichnet, dass** sie Geruchsdetektoren (15) aufweist, die am Umfang einer Anlage verteilt angeordnet sind, welche mindestens eine Quelle (10, 11, 12) übelriechender Gase aufweist, wobei diese Detektoren dazu angeordnet sind, um die Ergebnisse ihrer Messungen an eine Messungserfassungszentrale (B) zu übertragen, welche mit einer Steuereinheit (A) verbunden ist, welche, in Abhängigkeit von den Messergebnissen, mindestens ein Gebläse (8.1, 8.2, 8.3) aktiviert, um mindestens einen geeigneten Sektor (Sa1,...Sb3) von Injektoren zu versorgen.

3. Vorrichtung nach Anspruch 1 oder 2, **dadurch gekennzeichnet, dass** die Injektoren (5a, 5b) sich nahe dem oberen Rand der Geruchsquelle befinden.

4. Vorrichtung nach einem der vorhergehenden Ansprüche, **dadurch gekennzeichnet, dass** sie mindestens drei Injektorsektoren aufweist, die am Umfang einer oder jeder Quelle übelriechender Gerüche angeordnet sind, wobei jeder Sektor mindestens einen Injektor aufweist.

5. Vorrichtung nach einem der vorhergehenden Ansprüche, **dadurch gekennzeichnet, dass** jeder Winkelsektor (Sa1,...Sb3) und dessen Injektoren über eine Luftverteilerrampe versorgt sind, welche mit einem motorisch betätigten Ventil (13a1,...13b3) versehen ist, wobei die Rampe mit dem Auslass eines Gebläses (8a1, 8a2, 8a3) verbunden ist.

6. Vorrichtung nach einem der vorhergehenden Ansprüche, **dadurch gekennzeichnet, dass** die Injektoren (5a, 5b) in ihrer Ausrichtung verstellbar sind.

7. Vorrichtung nach einem der vorhergehenden Ansprüche, **dadurch gekennzeichnet, dass** die Einrichtungen (6a, 6b) zur Versorgung der Injektoren und die Steuereinrichtungen vorgesehen sind, um in Abhängigkeit von den atmosphärischen Bedingungen und den Geruchseinheitenmessungen einen variablen Druck zu gewährleisten.

8. Vorrichtung nach Anspruch 2, **dadurch gekennzeichnet, dass** die Steuereinheit (A) durch eine Verwaltungs- und Kontrollsoftware gesteuert ist, welche programmiert ist, um die Dispersionsparameter und die Geruchseinheitenmessungen in Echtzeit zu berücksichtigen.

9. Vorrichtung nach Anspruch 2 oder 8, **dadurch gekennzeichnet, dass** die Steuereinheit (A) durch eine prädiktive Simulationssoftware der natürlichen Dispersion der Geruchswolke (2) gesteuert ist, wobei die Software für eine präventive Aktion der automatischen Steuerung der Dispersionsvorrichtung durch Induktion integriert ist.

10. Vorrichtung nach einem der vorhergehenden Ansprüche, **dadurch gekennzeichnet, dass** sie eine Einrichtung zum Einblasen von "überdeckenden" Produkten in die Luft, welche die Injektoren speist, aufweist.

11. Vorrichtung nach einem der vorhergehenden Ansprüche, **dadurch gekennzeichnet, dass** die Einrichtungen zur Versorgung der Injektoren mindestens ein Gebläse (8.1, 8.2, 8.3) mit regelbarer Drehzahl und mindestens eine Verbindungsleitung zwischen dem Auslass des Gebläses und einer Rampe zur Versorgung des Injektors oder der Injektoren aufweist.

12. Vorrichtung nach Anspruch 11, **dadurch gekennzeichnet, dass** die Einrichtungen zur Versorgung des oder der Injektoren regelbar sind, um das Modulieren des Drucks und der an die Injektoren gelieferte Fördermenge an Luft oder Hilfsgas gemäß den momentanen atmosphärischen Bedingungen und den Eigenschaften der Gaswolke zu ermöglichen, wobei das Anhalten der Versorgungseinrichtungen gesteuert wird, wenn die natürliche Dispersion ausreichend ist.

## Claims

1. A device for dispersing foul gaseous effluent emissions at the source, **characterized in that** it comprises:
- a foul gaseous effluent source tank,
- at least two auxiliary gas or air injectors (5a, 5b) directed upward, installed at the periphery of the foul gaseous effluent source tank, in opposite zones, so as to disperse the foul gaseous effluents,
- means for supplying the injectors, each injector being supplied by a separate supply means,
- and control means sensitive to the wind direction arranged in order to select the start-up of the injector(s) best located, depending on the wind direction, for dispersing and/or raising the gaseous effluent plume.

2. The device as claimed in claim 1, **characterized in that** it comprises odor detectors (15) distributed at the periphery of a plant comprising at least one source (10, 11, 12) of foul odors, these detectors being arranged in order to communicate the results of their measurements to a central measurement acquisition station (B) connected to a control unit (A) which, depending on the results of the measurements, actuates at least one fan (8.1, 8.2, 8.3) in order to supply at least one appropriate section (Sa1...Sb3) of injectors.

3. The device as claimed in claim 1 or 2, **characterized in that** the injectors are located close to the upper edge of the source of odors.

4. The device as claimed in any one of the preceding claims, **characterized in that** it comprises at least three sections of injectors installed at the periphery of one, or of each, foul odor source, each section comprising at least one injector.

5. The device as claimed in any one of the preceding claims, **characterized in that** each angular section (Sa1,...Sb3), and its injectors, are supplied by an air manifold equipped with a motorized control valve (13a1,...13b3), the manifold being connected to the discharge of a fan (8a1, 8a2, 8a3).

6. The device as claimed in any one of the preceding claims, **characterized in that** the injectors have an adjustable orientation.

7. The device as claimed in any one of the preceding claims, **characterized in that** the means for supplying the injectors and the control means are provided to ensure a pressure that varies as a function of the atmospheric conditions and of odor unit measurements.

8. The device as claimed in claim 2, **characterized in that** the control unit (A) is driven by management and control software programmed to take into consideration the dispersion parameters and the odor unit measurements in real time.

9. The device as claimed in claim 2 or 8, **characterized in that** the control unit (A) is driven by simulation software that predicts the natural dispersion of the odor plume (2), which is integrated for a preventive action of automatically controlling the dispersion device by induction.

10. The device as claimed in any one of the preceding claims, **characterized in that** it comprises a means for injecting "masking" products into the air that supplies the injectors.

11. The device as claimed in any one of the preceding claims, **characterized in that** the means for supplying the injectors comprise at least one adjustable-speed fan (8.1, 8.2, 8.3), and at least one connection pipe between the outlet of the fan and a manifold of the injector(s).

12. The device as claimed in claim 11, **characterized in that** the means for supplying the injector(s) are adjustable in order to make it possible to regulate the pressure and the flow rate of auxiliary gas or air sent to the injectors depending on the atmospheric conditions of the moment, and the properties of the gas plume, the supply means being shut down when the natural dispersion is sufficient.
